# EUROPEAN PATENT APPLICATION

(11) **EP 4 085 857 A1**
(43) Date of publication of application: **09.11.2022**
(21) Application number: 22150966.4
(22) Date of filing: 11.01.2022
(51) Int. Cl.: A61B 18/08, A61B 90/00

(54) **THERMAL DEBRIDING TOOLS**

(30) Priority: 12.01.2021 US 202117146697
(71) Applicant: Medos International Sarl, 2400 Le Locle (CH)
(72) Inventor: BARRY, Donald E., Norwood, 02062 (US); LAPLACA, Matthew, Franklin, 02038 (US)
(74) Representative: Carpmaels & Ransford LLP

(57) **Abstract**

In general, thermal debriding tools and methods of using thermal debriding tools are provided. In an exemplary embodiment, a thermal debriding tool is configured to be advanced minimally invasively, e.g., arthroscopically, into a patient and to cut tissue using electrical energy. The thermal debriding tool includes a heating element configured to be positioned in contact with tissue and to be heated. The heated heating element is configured to cut the tissue so as to allow the thermal debriding tool to cut the tissue using electrical energy. The heating element can be a resistive heating element that is configured to become hot when a current is delivered to the heating element. The thermal debriding tool can include an actuator configured to be actuated to cause the current to be delivered to the heating element, thereby allowing the heating element to be heated on demand.

## Description

### FIELD

The present disclosure generally relates to thermal debriding tools and methods of using thermal debriding tools.

### BACKGROUND

The meniscus is specialized tissue found between the bones of a joint. For example, in the knee the meniscus is a C-shaped piece of fibrocartilage which is located at the peripheral aspect of the joint between the tibia and femur. This tissue performs important functions in joint health including adding joint stability, providing shock absorption, and delivering lubrication and nutrition to the joint. As a result, meniscal injuries can lead to debilitating conditions such as degenerative arthritis.

Meniscal injuries, and in particular tears, are a relatively common injury. Such injuries can result from a sudden twisting-type injury such as a fall, overexertion during a work-related activity, during the course of an athletic event, or in any one of many other situations and/or activities. In addition, tears can develop gradually with age. In either case, the tears can occur in either the outer thick part of the meniscus or through the inner thin part. While some tears may involve only a small portion of the meniscus, others affect nearly the entire meniscus.

Unfortunately, a damaged meniscus is unable to undergo the normal healing process that occurs in other parts of the body. The peripheral rim of the meniscus at the menisco-synovial junction is highly vascular (red zone) whereas the inner two-thirds portion of the meniscus is completely avascular (white zone), with a small transition (red-white zone) between the two. Degenerative or traumatic tears to the meniscus which result in partial or complete loss of function frequently occur in the white zone where the tissue has little potential for regeneration. Such tears result in severe joint pain and locking, and in the long term, a loss of meniscal function leading to osteoarthritis.

The majority of meniscal injuries are treated by removing the damaged tissue during a partial meniscectomy or, when the majority of the meniscal tissue is damaged, a total meniscectomy. However, mechanically removing meniscus tissue with a mechanical cutter is difficult due to the properties of the meniscus and the location of the meniscus. Mechanical cutters are slow to operate and may leave ragged tissue edges. A size of mechanical cutters is limited due to the location of the meniscus and due to a desire or need to use small portals to access the meniscus and cause minimum damage to surrounding tissue.

Accordingly, there remains a need for improved tissue removal tools.

### SUMMARY

In general, thermal debriding tools and methods of using thermal debriding tools are provided.

In one aspect, a surgical device is provided that in one embodiment includes a handle and an elongate shaft extending distally from the handle. The shaft is configured to be advanced arthroscopically into a patient. The surgical device also includes a conductive heating element at a distal end of the shaft. The heating element is configured to be heated such that contact of the heated heating element with tissue causes the heating element to cut the tissue. The surgical device also includes a conductive lead operably coupled to the actuator and to the heating element. The conductive lead extends through the shaft. The surgical device also includes an actuator configured to be actuated and thereby cause a current to be delivered along the conductive lead to the heating element, thereby heating the heating element.

The surgical device can have any number of variations. For example, the surgical device can also include an insulative guard extending distally from the elongate shaft, the insulative guard can include opposed distally-extending legs, and the heating element can be positioned between the distally-extending legs. In some embodiments, the insulative guard can be formed of a ceramic. In some embodiments, the insulative guard can be formed of a plastic. In some embodiments, the insulative guard can be formed of a ceramic and a plastic, the plastic can at least partially surrounds the ceramic, and the plastic can have a lower conductivity than the ceramic.

For another example, the surgical device can include a control circuit configured to cause the current delivered along the conductive lead to change during current delivery based on a resistance or temperature of the heating element. For still another example, the shaft can define a longitudinal axis, and the heating element can be U-shaped with opposed legs of the U-shape extending longitudinally and substantially parallel to the longitudinal axis. For yet another example, the heating element can include a substantially flat plate with a first edge of the plate facing distally and a second, opposite edge of the plate facing proximally. For still another example, the actuation of the actuator can be configured to cause the heating element to heat to a temperature in a range of about 500°C to about 1000°C.

For yet another example, the conductive lead can be formed of copper. In some embodiments, the heating element can be formed of a metal having a higher resistance than copper.

For still another example, an outer diameter of the shaft can be in a range of about 2 mm to about 5 mm. For another example, the tissue can be at one of a knee of the patient, a hip of the patient, and a shoulder of the patient. For yet another example, the tissue can include meniscus tissue.

In another aspect, a surgical method is provided that in one embodiment includes arthroscopically advancing a surgical device to a knee of a patient. The surgical device includes a handle and an elongate shaft extending distally from the handle. The shaft is configured to be advanced arthroscopically into the patient. The surgical device also includes a conductive heating element at a distal end of the shaft. The heating element is configured to be heated such that contact of the heated heating element with tissue causes the heating element to cut the tissue. The surgical device also includes a conductive lead operably coupled to the actuator and to the heating element. The conductive lead extends through the shaft. The surgical device also includes an actuator configured to be actuated and thereby cause a current to be delivered along the conductive lead to the heating element, thereby heating the heating element. The surgical method also includes actuating the actuator, thereby causing the heating element to be heated and cut meniscus tissue.

The surgical method can have any number of variations. For example, the surgical device can also include an insulative guard extending distally from the elongate shaft, the insulative guard can include opposed distally-extending legs, and the heating element can be positioned between the distally-extending legs. In some embodiments, the insulative guard can be formed of a ceramic. In some embodiments, the insulative guard can be formed of a plastic. In some embodiments, the insulative guard can be formed of a ceramic and a plastic, the plastic can at least partially surrounds the ceramic, and the plastic can have a lower conductivity than the ceramic.

For another example, the surgical device can include a control circuit configured to cause the current delivered along the conductive lead to change during current delivery based on a resistance or temperature of the heating element. For still another example, the shaft can define a longitudinal axis, and the heating element can be U-shaped with opposed legs of the U-shape extending longitudinally and substantially parallel to the longitudinal axis. For yet another example, the heating element can include a substantially flat plate with a first edge of the plate facing distally and a second, opposite edge of the plate facing proximally. For still another example, the actuation of the actuator can be configured to cause the heating element to heat to a temperature in a range of about 500°C to about 1000°C.

For yet another example, the conductive lead can be formed of copper. In some embodiments, the heating element can be formed of a metal having a higher resistance than copper.

For still another example, an outer diameter of the shaft can be in a range of about 2 mm to about 4 mm. For another example, the tissue can be at one of a knee of the patient, a hip of the patient, and a shoulder of the patient. For yet another example, the tissue can include meniscus tissue.

In another embodiment, a surgical method is provided that includes arthroscopically advancing a surgical device to one of a hip and a shoulder of a patient. The surgical device includes a handle and an elongate shaft extending distally from the handle. The shaft is configured to be advanced arthroscopically into the patient. The surgical device also includes a conductive heating element at a distal end of the shaft. The heating element is configured to be heated such that contact of the heated heating element with tissue causes the heating element to cut the tissue. The surgical device also includes a conductive lead operably coupled to the actuator and to the heating element. The conductive lead extends through the shaft. The surgical device also includes an actuator configured to be actuated and thereby cause a current to be delivered along the conductive lead to the heating element, thereby heating the heating element. The surgical method also includes actuating the actuator, thereby causing the heating element to be heated and cut tissue.

The surgical method can have any number of variations. For example, the surgical device can also include an insulative guard extending distally from the elongate shaft, the insulative guard can include opposed distally-extending legs, and the heating element can be positioned between the distally-extending legs. In some embodiments, the insulative guard can be formed of a ceramic. In some embodiments, the insulative guard can be formed of a plastic. In some embodiments, the insulative guard can be formed of a ceramic and a plastic, the plastic can at least partially surrounds the ceramic, and the plastic can have a lower conductivity than the ceramic.

For another example, the surgical device can include a control circuit configured to cause the current delivered along the conductive lead to change during current delivery based on a resistance or temperature of the heating element. For still another example, the shaft can define a longitudinal axis, and the heating element can be U-shaped with opposed legs of the U-shape extending longitudinally and substantially parallel to the longitudinal axis. For yet another example, the heating element can include a substantially flat plate with a first edge of the plate facing distally and a second, opposite edge of the plate facing proximally. For still another example, the actuation of the actuator can be configured to cause the heating element to heat to a temperature in a range of about 500°C to about 1000°C.

For yet another example, the conductive lead can be formed of copper. In some embodiments, the heating element can be formed of a metal having a higher resistance than copper.

For still another example, an outer diameter of the shaft can be in a range of about 2 mm to about 4 mm. For another example, the tissue can be at one of a knee of the patient, a hip of the patient, and a shoulder of the patient. For yet another example, the tissue can include meniscus tissue.

In another embodiment, a surgical method includes positioning a conductive heating element of an arthroscopic surgical tool in contact with tissue of a patient. The heating element is at a distal end of an elongate shaft of the surgical tool. The surgical method also includes causing a current to be conducted through a conductive lead extending through the elongate shaft, thereby heating the heating element such that the heated heating element cuts the tissue.

The surgical method can vary in any number of ways. For example, the surgical method can include, using a control circuit of the surgical tool, causing the current conducted through the conductive lead to change during conduction of the current based on a resistance or temperature of the heating element, and heating the heating element can include heating the heating element to heat to a temperature in a range of about 500°C to about 1000°C.

For another example, an insulative guard can extend distally from the elongate shaft and include opposed distally-extending legs, the heating element can be positioned between the distally-extending legs, and the insulative guard can protect tissue and/or other material that is adjacent to the tissue being cut from being contacted by or heated by the heated heating element. In some embodiments, the insulative guard can be formed of a ceramic. In some embodiments, the insulative guard can be formed of a plastic. In some embodiments, the insulative guard can be formed of a ceramic and a plastic, the plastic can at least partially surrounds the ceramic, and the plastic can have a lower conductivity than the ceramic.

For yet another example, causing the current to be conducted through the conductive lead can include actuating an actuator of the surgical tool. For still another example, the conductive lead can be formed of copper, and the heating element can be formed of a metal having a higher resistance than copper. For another example, the surgical method can also include advancing the surgical tool into the patient, and the tissue can include meniscus tissue. For yet another example, the tissue can be at one of a knee of the patient, a hip of the patient, and a shoulder of the patient. For still another example, an outer diameter of the shaft can be in a range of about 2 mm to about 4 mm. For another example, the shaft can define a longitudinal axis, and the heating element can be U-shaped with opposed legs of the U-shape extending longitudinally and substantially parallel to the longitudinal axis. For yet another example, the heating element can include a substantially flat plate with a first edge of the plate facing distally and a second, opposite edge of the plate facing proximally. For another example, heating the heating element can include heating the heating element to heat to a temperature in a range of about 500°C to about 1000°C.

### BRIEF DESCRIPTION OF DRAWINGS

This disclosure will be more fully understood from the following detailed description taken in conjunction with the accompanying drawings, in which:
FIG. 1 is a perspective view of meniscus tissue cut using a mechanical biter;
FIG. 2 is a perspective view of meniscus tissue cut using a thermal debriding tool;
FIG. 3 is a side schematic view of one embodiment of a thermal debriding tool;
FIG. 4 is a circuit diagram of a control circuit of the tool of FIG. 3;
FIG. 5 is a side schematic view of another embodiment of a thermal debriding tool;
FIG. 6 is a perspective view of a distal portion of the tool of FIG. 5 including one embodiment of a heating element;
FIG. 7 is a perspective view of a distal portion of the tool of FIG. 5 including another embodiment of a heating element;
FIG. 8 is a cross-sectional view of the tool of FIG. 7;
FIG. 9 is a distal end view of the heating element of FIG. 7;
FIG. 10 is a distal end view of another embodiment of a heating element;
FIG. 11 is a perspective view of a distal portion of the tool of FIG. 5 including yet another embodiment of a heating element;
FIG. 12 is a cross-sectional view of the tool of FIG. 11;
FIG. 13 is a cross-sectional view of a distal portion of the tool of FIG. 5 including still another embodiment of a heating element;
FIG. 14 is a perspective view of another embodiment of a heating element;
FIG. 15 is a front view of a knee of a patient with the tool of FIG. 3 and with one embodiment of an arthroscope advanced into the patient;
FIG. 16 is a side schematic view of the tool of FIG. 15 positioned relative to a meniscus tissue of the patient; and
FIG. 17 is a side schematic view of the meniscus tissue of FIG. 16 after being cut by the tool of FIG. 16.

### DETAILED DESCRIPTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices, systems, and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. Sizes and shapes of the systems and devices, and the components thereof, can depend at least on the anatomy of the subject in which the systems and devices will be used, the size and shape of components with which the systems and devices will be used, and the methods and procedures in which the systems and devices will be used.

In general, thermal debriding tools and methods of using thermal debriding tools are provided. In an exemplary embodiment, a thermal debriding tool is configured to be advanced minimally invasively, e.g., arthroscopically, into a patient and to cut tissue using electrical energy. The thermal debriding tool includes a heating element configured to be positioned in contact with tissue and to be heated. The heated heating element is configured to cut the tissue so as to allow the thermal debriding tool to cut the tissue using electrical energy. The heating element can be a resistive heating element that is configured to become hot when a current is delivered to the heating element. The thermal debriding tool can include a conductive lead configured to deliver the current to the heating element. The thermal debriding tool can include an actuator configured to be actuated to cause the current to be delivered along the conductive lead to the heating element, thereby allowing the heating element to be heated on demand. The actuator can also be configured to be actuated again to stop the current delivery to the heating element, thereby allowing the heating element to cool down after being heated. Allowing the heating element to cool may prevent the heating element from damaging tissue and/or other material during removal of the thermal debriding tool from a patient's body.

The thermal debriding tool can include a control circuit configured to change the current delivered to the heating element in real time with the cutting of the tissue. Changing the current allows for the heating element's temperature to be controlled so that the heating element does not become too hot, which may result in too much tissue being cut and/or structure(s) adjacent to the heating element becoming undesirably heated, and so that the heating element does not become too cool, which may result in the heating element not being hot enough to cut tissue. The heating element may lower in temperature during cutting due to exposure to a liquid present at the surgical site. In some surgical procedures, such as meniscal debridement or repair procedures, the surgical site has saline and/or other liquid delivered to the surgical site to improve visualization at the surgical site. The liquid can cause the heating element to drop in temperature if the current being delivered to the heating element remains constant. The control circuit being able to control the current delivered to the heating element, e.g., increase the amount of current to increase the heating element's temperature, in real time with the current delivery may offset the cooling effects of the liquid to keep the heating element at a temperature effective for cutting tissue.

The control circuit can be configured to change the current delivered to the heating element based on a resistance or temperature of the heating element. In general, electrical resistance of a material changes (increases or decreases) when temperature changes (increases or decreases). A temperature coefficient of a material numerically reflects the change in the material's resistance with temperature. Materials have known temperature coefficients, so the material of the heating element will have a known temperature coefficient. The control circuit can be configured to monitor the resistance of the heating element and to adjust the heating element's temperature based on the monitoring. The control circuit can thus be configured to adjust the heating element's temperature by changing the current delivered to the heating element. The current can be changed in real time with the delivery of the current to the heating element, and thus in real time with the heating element cutting tissue, which may provide for consistent, effective cutting of the tissue.

The thermal debriding tools described herein are configured to be used in a meniscal debridement or repair procedure in which meniscus tissue is cut. The thermal debriding tool can thus be used to cut meniscus tissue using electrical energy.

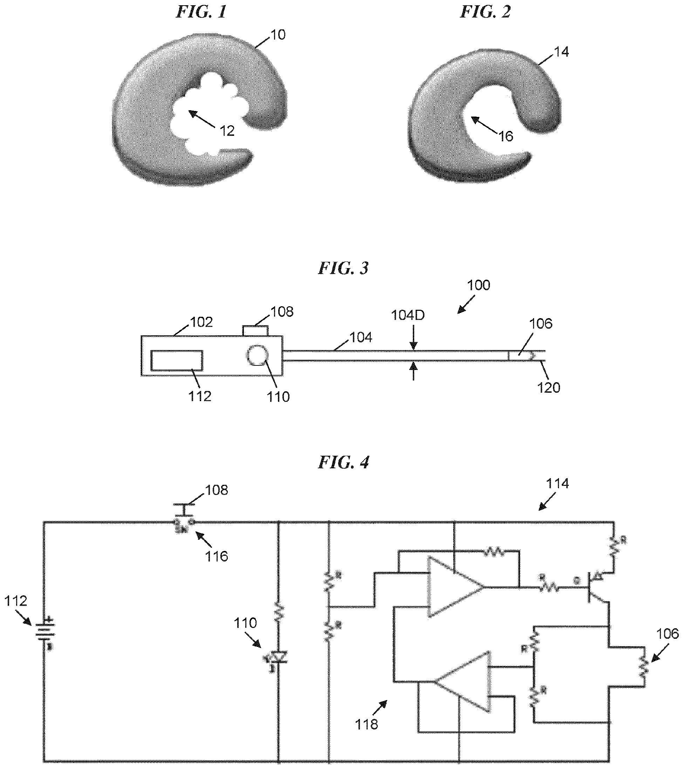

Meniscus tissue is traditionally cut mechanically in meniscal debridement or repair procedures using mechanical cutting tools, such as biters, punchers, scissors, and scalpels, that can be powered or unpowered. Meniscus tissue is dense and is therefore difficult to cut mechanically even with a very sharp mechanical cutting tool. Mechanical cutting tools are slow to operate because each cut of the tissue is separately made with the cutting tool being repositioned between each cut. Mechanical cutting tools leave ragged tissue edges due to the need to remove small tissue pieces with each cut and because mechanical cutting tools tend to pull on the tissue being cut. Ragged tissue edges are susceptible to continued tearing after completion of the surgical procedure, which may cause patient pain and the need for another surgical procedure to be performed. FIG. 1 illustrates one embodiment of meniscus tissue 10 having been cut a plurality of times using a mechanical biter, leaving ragged tissue edges 12. Additionally, the size of mechanical cutting tools is limited due the desire to cause minimum damage to surrounding tissue, in minimally invasive surgical procedures the need to use small portals, and in meniscus treatment at the knee the location of the meniscus. Smaller mechanical cutting tools generally make smaller individual cuts than larger mechanical cutting tools, thereby increasing a total number of cuts that need to be made and thus a length of time needed to cut tissue. The mechanical strength of small mechanical cutting tools is limited due to the high forces on pivots and cutting surfaces, which can also make cutting dense meniscus tissue difficult.

Cutting meniscus tissue using a thermal debriding tool that cuts the meniscus tissue with electrical energy may provide one or more benefits as compared to cutting meniscus tissue using a mechanical cutting tool. The thermal debriding tool is configured to be guided along the edge of the meniscus tissue to cut the tissue by applying electrical energy, e.g., heat, to the tissue. The heat applied to the tissue melts and seals the tissue as the heating element cuts the tissue. The thermal debriding tool does not pull on the tissue being cut as the tool is moved along the tissue. The cut tissue edge can thus be smooth instead of being ragged, thereby reducing the potential for continued tearing of the tissue after completion of the surgical procedure. FIG. 2 illustrates one embodiment of meniscus tissue 14 having been cut a using a thermal debriding tool, leaving a smooth tissue edge 16. The thermal debriding tool can be continuously guided along the edge of the meniscus tissue so as to not require repeated repositioning of the thermal debriding tool during tissue cutting as would be needed to cut the same amount of tissue using a mechanical cutting tool, which may shorten the amount of time needed to cut the tissue and/or may reduce surgeon hand strain. Any frayed or ragged tissue edges that are present can be smoothed using the thermal debriding tool. Using electrical energy to cut the tissue allows for a smaller diameter tool, as compared to a mechanical cutting tool, because the loading forces can be much lower by using electrical energy than when using mechanical force to cut tissue. The thermal debriding tool can be sized to access the meniscus tissue in a minimally invasive surgical procedure similar to a mechanical cutting tool, e.g., by being advanced through an arthroscopic portal to the meniscus tissue, so as to be a familiar surgical access technique to surgeons while providing the benefit(s) of cutting tissue using electrical energy, such as those discussed herein, that cannot be achieved by cutting tissue mechanically.

The thermal debriding tools described herein can be used in meniscal debridement or repair procedures that treat meniscus tissue at a knee, hip, or shoulder and can be used in other surgical procedures in which tissue needs to be cut. Similar benefits can be achieved in these surgical procedures as discussed herein with respect to meniscal debridement or repair procedures, e.g., smooth tissue edges, smaller diameter tools, etc.

FIG. 3 illustrates one embodiment of a thermal debriding tool 100 configured to be advanced minimally invasively into a patient and to cut tissue using electrical energy. The tool 100 includes a handle 102, an elongate shaft 104 extending distally from the handle 102, and a heating element 106 at a distal end of the shaft 104.

The handle 102 is configured to be handheld by a user, e.g., a surgeon or other medical professional. In some embodiments the handle 102 can instead be manipulated by a robotic surgical system. The handle 102 has a substantially rectangular shape in this illustrated embodiment but can have any of a variety of shapes. A person skilled in the art will appreciate that a shape may not be precisely rectangular but nevertheless be considered to be substantially rectangular due to any number of factors, such as manufacturing tolerances and sensitivity of measurement equipment.

The shaft 104 is configured to be advanced minimally invasively into a patient, such as advanced arthroscopically into a patent in an arthroscope surgical procedure. The shaft 104 has an outer diameter 104D of a size to facilitate the shaft's minimally invasive use. In an exemplary embodiment, the outer diameter 104D of the shaft 104 is in a range of about 2 mm to about 5 mm. A person skilled in the art will appreciate that a value may not be precisely at a value but nevertheless be considered to be about that value due to any number of factors, such as manufacturing tolerances and sensitivity of measurement equipment. In some embodiments, the outer diameter 104D of the shaft 104 can be about 2 mm, which is equivalent to a 15 to 14 gauge needle. In some embodiments, the outer diameter 104D of the shaft 104 is in a range of about 2 mm to about 4 mm. In some embodiments, the outer diameter 104D of the shaft 104 is in a range of about 4 mm to about 5 mm.

The heating element 106 is configured to be positioned in contact with tissue and to be heated. The heated heating element 106 is configured to cut the tissue so as to allow the thermal debriding tool 100 to cut the tissue using electrical energy. In an exemplary embodiment, the heating element 106 is configured to be heated to a temperature in a range of about 500°C to about 1000°C. The heating element 106 being heated to a temperature in a range of about 500°C to about 1000°C can allow the heating element 106 to cut tissue. A lower temperature, such as a temperature in a range of about 60°C to about 80°C, allows a heating element to seal tissue by melting tissue proteins, but the heating element must be hotter in order to cut tissue.

The heating element 106 is conductive. The heating element 106 is thus formed of a conductive material and is a resistive heating element. Being a resistive heating element, current applied to the heating element 106 is configured to heat the heating element 106. Examples of conductive materials that can be used to form the heating element 106 include nichrome, Kanthal^{®} (iron-chromium-aluminium (FeCrAl) alloys), NiFe30, tungsten, and steels such as SS304 (American Iron and Steel Institute (AISI) 304 grade stainless steel).

The tool 100 includes an actuator 108 at the handle 102 that is configured to be actuated to cause current to be delivered to the heating element 106. The actuator 108 is configured to move between an unactuated position, in which current is not being delivered to the heating element 106, and an actuated position, in which current is being delivered to the heating element 106. The actuator 108 is in the unactuated positon in FIG. 3. The actuator 108 is a depressible button in this illustrated embodiment but can have other configurations, such as a lever or a rotatable knob. Depressing the button 108 causes the actuator 108 to move from the unactuated position to the actuated position. Releasing the button 108 causes the actuator 108 to move from the actuated position to the unactuated position. In embodiments in which the actuator is a lever, the actuator can be configured to move between the unactuated and actuated positions, for example, by pivoting the lever between positions corresponding to the unactuated and actuated positions. In embodiments in which the actuator is a rotatable knob, the actuator can be configured to move between the unactuated and actuated positions, for example, by rotating the knob in opposite directions to cause movement between the unactuated and actuated positions, e.g., rotating in one of clockwise and counterclockwise to be in the actuated position and the other of clockwise and counterclockwise to be in the unactuated position.

The tool 100 includes an indicator light 110 at the handle 102 that is configured to indicate to a user whether current is being delivered to the heating element 106. The indicator light 110 is thus configured to indicate whether the actuator 108 is in the actuated position (heat is being delivered to the heating element 106) or is in the unactuated position (heat is not being delivered to the heating element 106). The indicator light 110 includes a light-emitting diode (LED) light in this illustrated embodiment, other types of lights can be alternatively or additionally used. The indicator light 110 is configured to be on (emitting light) when heat is being delivered to the heating element 106 and to be off (not emitting light) when heat is not being delivered to the heating element 106. In other embodiments, the indicator light 110 can be configured to be off when the actuator 108 is in the unactuated position and to be on when the actuator 108 is in the actuated position.

Instead of or in addition to the indicator light 110, the tool 100 can include an indicator that is not a light and that is configured to indicate to a user whether current is being delivered to the heating element 106. For example, the indicator can include a window formed in the handle 102 that is configured to show a first color therein when the actuator 108 is in the actuated position (heat is being delivered to the heating element 106) and a second, different color therein when the actuator 108 is in the unactuated position (heat is not being delivered to the heating element 106). The actuation of the actuator 108 can be configured to cause mechanical movement of a plate or other element in the handle 102, with the first color on the plate being visible through the window when the actuator 108 is in the actuated position (heat is being delivered to the heating element 106) and the second color on the plate being visible through the window when the actuator 108 is in the unactuated position (heat is not being delivered to the heating element 106). Instead of or in addition to different colors, a symbol, text, etc. can be shown in the window to indicate to a user whether current is being delivered to the heating element 106.

The tool 100 includes a power source 112 at the handle 102 that is configured to provide power to the indicator light 110 and to supply current to be delivered to the heating element. The power source 112 includes a battery in this illustrated embodiment, but other types of power sources can alternatively or additionally be used.

The tool 100 includes a control circuit 114 (see FIG. 4) at the handle 102. The control circuit 114 includes a switch 116 configured to operatively engage the actuator 108. With the actuator 108 in the unactuated position, as shown in FIGS. 3 and 4, the switch 116 is open. With the switch 116 open, the power source 112 is not providing power to the indicator light 110, such that the indicator light is off, and current is not being and cannot be delivered to the heating element 106, such that the heating element 106 is not being heated. With the actuator 108 in the actuated position, the switch 116 is closed. With the switch 116 closed, the power source 112 is providing power to the indicator light 110, such that the indicator light is on, and current is being delivered to the heating element 106, such that the heating element 106 is being heated. The actuator 108 being actuated, e.g., the button being depressed, the lever being moved, the knob being rotated, etc., is thus configured to cause the switch 116 to move from being open to being closed. Subsequent actuation of the actuator 108, e.g., the button being released, the lever being moved in an opposite direction, the knob being rotated in an opposite direction, etc., is configured to cause the switch 116 to move from being closed to being open. The actuator 108 can be actuated and unactuated any number of times during use of the tool 100.

The control circuit 114 is configured to cause the current delivered to the heating element 108 to change during current delivery, e.g., when the actuator 108 is in the actuated positon and the switch 116 is closed. The current delivered to the heating element 106 can thus dynamically change during heating of the heating element 106. The delivered current changing during current delivery can help maintain the temperature of the heating element 106. As discussed above, maintaining the heating element's temperature at a substantially constant predetermined temperature or within a predetermined temperature range can help prevent the heating element 106 from becoming too hot or too cool. A person skilled in the art will appreciate that a value may not be precisely at a value but nevertheless be considered to be about that substantially at that value due to any number of factors, such as manufacturing tolerances and sensitivity of measurement equipment. In an exemplary embodiment, the heating element 106 is maintained at a temperature in a range of about 500°C to about 1000°C. As mentioned above, in some surgical procedures, such as meniscal debridement or repair procedures, the surgical site has saline and/or other liquid delivered to the surgical site. The liquid can result in the environment near but not in contact with the heating element 106 not being heated nearly as much as the heating element 106, which may help prevent the heating element 106 from damaging any tissue and/or other materials other than the intended tissue to be cut. For example, if the heating element 106 is heated to about 500°C, the environment near but not in contact with the heating element 106 can be heated to a maximum of about 100°C due to the quenching effect of the surrounding liquid. If there is sufficient quantity of liquid the heat capacity of the liquid will rapidly cool the surrounding area to the ambient temperature of the liquid, about 24°C, which is below body temperature (about 37°C) and thus unlikely to cause tissue damage.

The control circuit 114 is configured to cause the current delivered to the heating element 108 to change during current delivery based on a resistance of the heating element 106. The control circuit 114 includes an op amp circuit 118 configured to detect a voltage drop across the heating element 106, e.g., across the resistance of the heating element 106, to control the current delivery and thereby control the temperature of the heating element 106. Thus, as shown in FIG. 4, the tool 100 can include a closed loop system in which the current delivered to the heating element 106 is controlled such that the temperature of the heating element 106 is controlled.

The control circuit 114 can be constructed in any of a variety of ways, such as using a printed circuit board (PCB).

The current can be delivered to the heating element 106 in a variety of ways. In an exemplary embodiment, the shaft 104 can be conductive, e.g., made from a conductive material such as stainless steel or other conductive material, and can thus be configured to conduct the current to the heating element 106. Using the shaft 104 as a conductor to deliver current to the heating element 106 takes advantage of the tool 100 already including the shaft 104. Using the shaft 104 to deliver current to the heating element 106 may help allow for a small outer diameter 104D because an element to conduct the current need not be disposed inside of the shaft 104.

In another exemplary embodiment, the shaft 104 in combination with a single conductive lead, e.g., a wire, a cable, tape, etc., disposed in and extending through the shaft 104 can be configured to deliver the current to the heating element 106. Using a single conductive lead in combination with the shaft 104 to deliver current to the heating element 106 may help prevent the current-delivering shaft 104 from causing any damage to the shaft 104 and/or structure(s) adjacent to the shaft 104.

In yet another exemplary embodiment, a conductive lead, e.g., a wire, a cable, tape, etc., including a pair of conductive leads can be disposed in and extend through the shaft 104 and can be configured to deliver the current to the heating element 106. Using a conductive lead as a conductor to deliver current to the heating element 106 without using the shaft 104 as part of the conductor may allow for the shaft 104 to be formed of a non-conductive material or a material with relatively poor conductivity. Using a conductive lead as the current conductor instead of using the shaft 104 as the current conductor keeps current conduction occurring within the shaft 104 and thus may help prevent the current being delivered along the shaft 104 and causing any damage to the shaft 104 and/or structure(s) adjacent to the shaft 104.

In an exemplary embodiment, the conductor used to deliver current to the heating element 106 has a lower resistance than the heating element 106. The heat can therefore be concentrated at the heating element 106 instead of along the conductor. In embodiments using at least one conductive lead, the at least one conductive lead can be formed of copper, and the heating element 106 can be formed of a metal having a higher resistance than copper.

As shown in FIG. 3, the tool 100 includes an insulative guard 120. The insulative guard 120 is configured as an insulator to help keep the heating element's heat concentrated at the heating element 106 and to help prevent the heated heating element 106 from contacting tissue and/or other structures not intended to be cut.

The insulative guard 120 can be formed of a non-conductive, insulative material to facilitates the insulative guard's functionality as an insulator. In an exemplary embodiment, the insulative guard 120 is formed of a ceramic. In another exemplary embodiment, the insulative guard 120 is formed of a plastic. In yet another exemplary embodiment, the insulative guard 120 can be a multi-layer member formed of multiple materials. Each of the materials can have a different conductivity, which may help reduce heat the farther away from the heating element 106. For example, the insulative guard 120 can be formed of a ceramic and a plastic, with the plastic at least partially surrounding the ceramic and with the plastic having a lower conductivity than the ceramic. The insulative guard 120 can thus have a shell of lower conductivity material, e.g., plastic, at least partially surrounding a higher conductivity material, e.g., ceramic, that is closer to the heating element 106 than the lower conductivity material.

The insulative guard 120 extends distally from the elongate shaft 104 distally beyond the heating element 106. The insulative guard 120 can, as shown in FIG. 3, define a pair of opposed legs that extend distally beyond the heating element 106 on opposed sides of the heating element 106. The opposed legs can allow for the heating element 106 to come into contact with tissue to cut the tissue while protecting nearby tissue and/or other structures from being contacted by the heating element 106. In surgical procedures in which the heating element 106 is used near cartilage, such as in meniscal debridement or repair procedures, the insulative guard 120 may be particularly useful since cartilage is particularly susceptible to heat damage.

In an exemplary embodiment, the tool 100 is configured for single patient use and to be disposable.

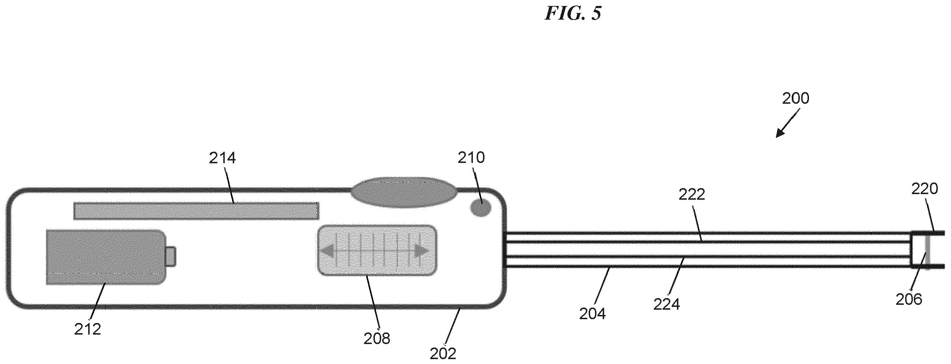

FIG. 5 illustrates another embodiment of a thermal debriding tool 200 configured to be advanced minimally invasively into a patient and to cut tissue using electrical energy. The tool 200 is generally configured and used similar to the thermal debriding tool 100 of FIG. 3 and includes a handle 202, an elongate shaft 204 extending distally from the handle 202, a heating element 206 at a distal end of the shaft 204, an actuator 208, an indicator light 210, a power source 212, a control circuit 214, and an insulative guard 220. The actuator 208 in this illustrated embodiment is in the form of a rotatable knob.

The tool 200 in this illustrated embodiment includes a pair of conductive leads 222, 224 configured to deliver current to the heating element 206, disposed in and extending through the shaft 204, and operatively coupled to the heating element 206 and the control circuit 214.

The tool 200 in this illustrated embodiment includes a second actuator 226 configured to be actuated to turn the tool 200 on, e.g., to activate the power source 212. The second actuator 226 is configured to move between a first position, in which the power source 212 is not providing power to the circuit board 214, and a second position, in which the power source is providing power to the circuit board 214. The second actuator 226 is in the first position in FIG. 5. The tool 200 including the second actuator 226 may help prevent the power source 212 from being depleted of power, e.g., a battery running dry, etc., before the tool 200 is finished being used. The second actuator 226 may serve as a safety feature. Allowing power to be controlled via the second actuator 226 may help prevent accidental heating of the heating element 206, e.g., if the actuator 208 is accidentally actuated before the heating element 206 is desired to be heated. The second actuator 226 is a depressible button in this illustrated embodiment but can have other configurations, such as a lever or a rotatable knob. Depressing the button 226 causes the second actuator 226 to move from the first position to the second position. Depressing the button 226 again causes the second actuator 226 to move from the second position to the first position.

In an exemplary embodiment, the tool 200 is configured for single patient use and to be disposable.

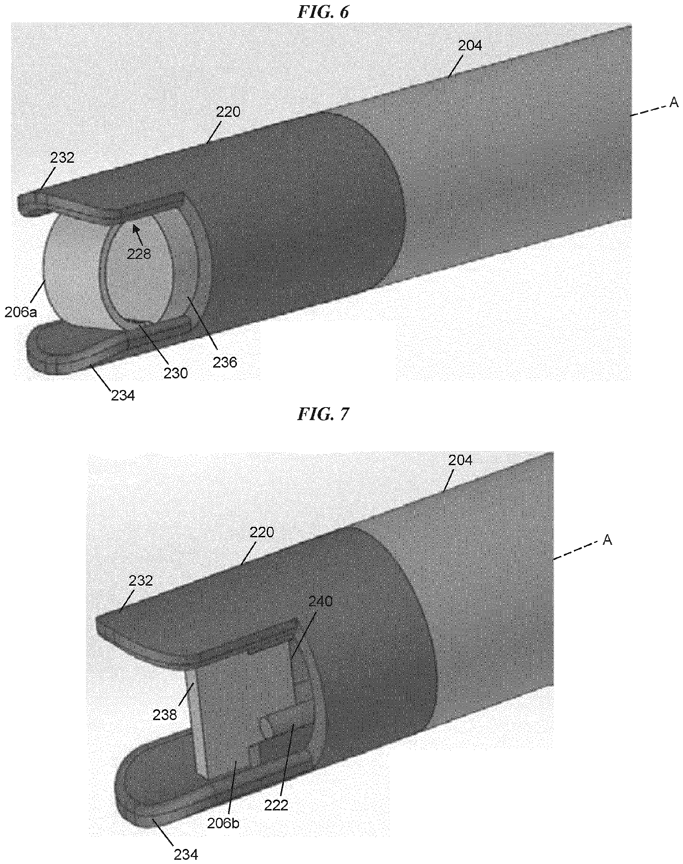

FIG. 6 illustrates one embodiment of the heating element 206 in which the heating element 206a has a U-shape. Opposed legs 228, 230 of the U-shaped heating element 206a extend longitudinally and substantially parallel to a longitudinal axis A of the shaft 204, which may facilitate attachment of the heating element 206a to the tool 200. A person skilled in the art will appreciate that an element, e.g., the legs 228, 230, may not be precisely parallel to another element, e.g., the longitudinal axis A, but nevertheless be considered to be substantially parallel to the other element for any of a variety of reasons, such as manufacturing tolerances and sensitivity of measurement equipment. The curved bottom of the U-shape located between the legs 228, 230 faces distally, which may facilitate contact of tissue to be cut with the curved bottom of the U-shape. The curved shape of the heating element 206a may facilitate smooth cutting of tissue as the heating element 206a, when heated, is moved along the tissue to cut the tissue.

FIG. 6 also illustrates opposed distal legs 232, 234 of the insulative guard 220 extending distally beyond the heating element 206a. The insulative guard 220 shares the longitudinal axis A with the shaft 204. The opposed distal legs 232, 234 of the insulative guard 220 are substantially parallel to one another and to the longitudinal axis A.

As shown in FIG. 6, the tool 200 includes a connector 236 configured to facilitate delivery of the current from the conductive leads 222, 224 to the heating element 206a. The connector 236 is conductive, e.g., formed of a conductive material. The conductive leads 222, 224 are attached to the connector 236, which is attached to the heating element 206a. The current delivered along the conductive leads 222, 224 can thus pass from the conductive leads 222, 224 to the connector 236 and from the connector 236 to the heating element 206a.

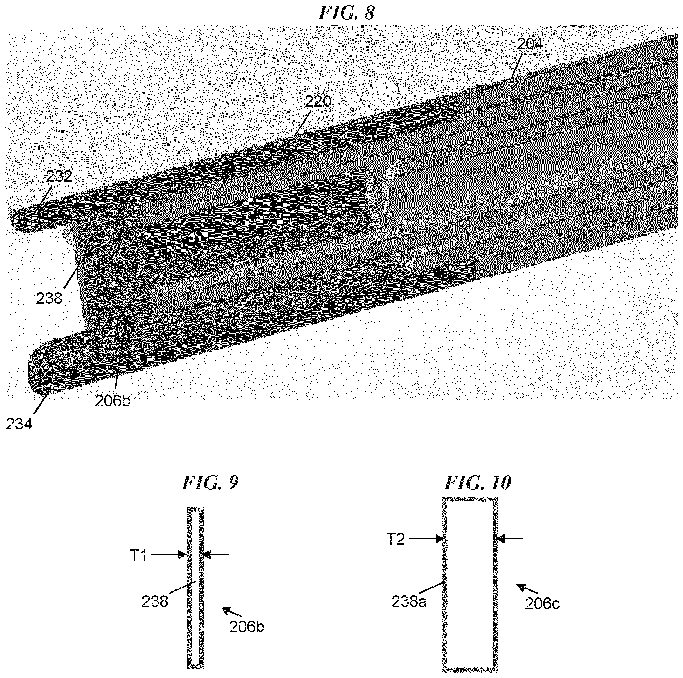

FIGS. 7 and 8 illustrate another embodiment of the heating element 206 in which the heating element 206b includes a substantially flat plate. A first edge 238 of the plate faces distally and a second, opposite edge 240 of the plate faces proximally. FIG. 9 illustrates a thickness T1 of the heating element 206b. The distal-facing first edge 238 is configured as a scraper that scrapes tissue when the first edge 238 is moved along an edge of the tissue. Thus, when heated, the heated heating element 206b can be moved along the edge of the tissue to scrape away tissue at the edge. The heating element 206b including a substantially flat plate allows the distal-facing first edge 238 to be substantially straight and substantially flat, which may help a surgeon or other user predictably determine where the heating element 206b will contact and cut tissue, even if the first edge 238 is only partially visible or is not visible at all, because of the substantially straight, substantially flat configuration of the heating element 206b. The substantially flat plate may thus facilitate precise cutting of the tissue. FIGS. 7 and 8 also illustrate the opposed distal legs 232, 234 of the insulative guard 220 extending distally beyond the heating element 206b.

FIG. 10 illustrates another embodiment of the heating element 206 in which the heating element 206c includes a substantially flat plate. The embodiment of FIG. 10 is the same as the embodiment of FIGS. 7 and 8 except that the heating element 206c of FIG. 10 has a thickness T2 that is greater than the thickness T1 of the heating element 206c of FIGS. 7-9. Other thicknesses of substantially flat plates are possible, such as a thickness that is greater than the thickness T1 of FIG. 9 and less than the thickness T2 of FIG. 10, a thickness that is greater than the thickness T2 of FIG. 10, etc.

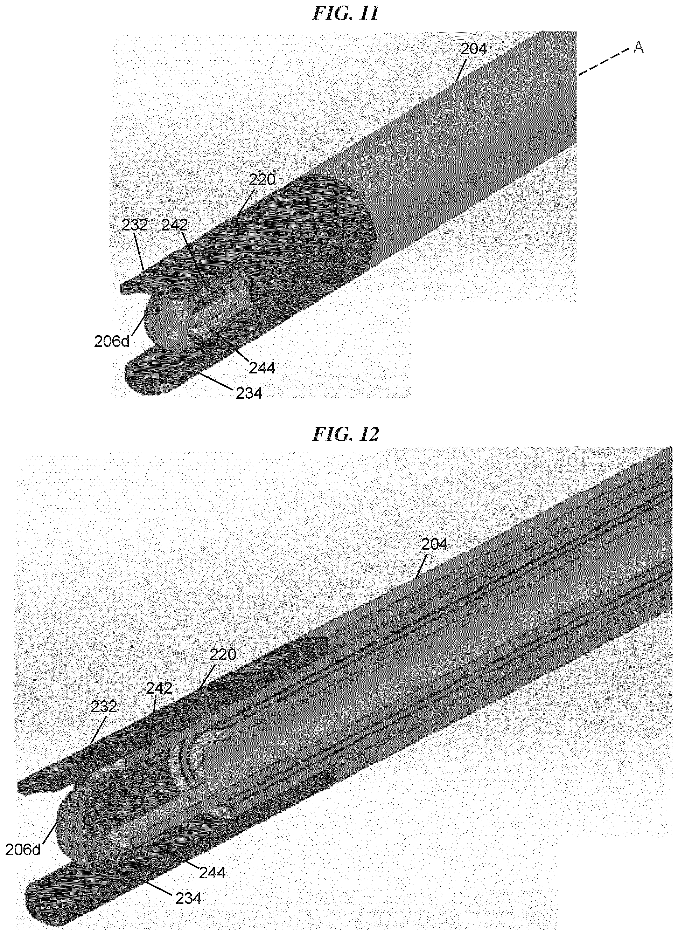

FIGS. 11 and 12 illustrate another embodiment of the heating element 206 in which the heating element 206d has a U-shape. Opposed legs 242, 244 of the U-shaped heating element 206d extend longitudinally and substantially parallel to the longitudinal axis A of the shaft 204 and the insulative guard 220, which may facilitate attachment of the heating element 206d to the tool 200. The curved bottom of the U-shape located between the legs 242, 244 faces distally, which may facilitate contact of tissue to be cut with the curved bottom of the U-shape. The curved shape of the heating element 206d may facilitate smooth cutting of tissue as the heating element 206d, when heated, is moved along the tissue to cut the tissue. FIGS. 11 and 12 also illustrate the opposed distal legs 232, 234 of the insulative guard 220 extending distally beyond the heating element 206d.

The curved bottom of the U-shaped heating element 206d of FIGS. 11 and 12 has a spherical shape. The U-shaped heating element 206a of FIG. 6 does not have a spherical shape. Instead, the U-shaped heating element 206a is configured as a substantially flat plate that is molded in or bent into U-shape. The spherical heating element 206d of FIGS. 11 and 12 allows the heating element 206d to protrude distally more than the heating element 206a of FIG. 6 protrudes distally, which may allow for more contact of the heating element 206d (FIGS. 11 and 12) with tissue as compared to the heating element 206a (FIG. 6). The spherical shape of FIGS. 11 and 12 also provides more surface area than the non-spherical shape of FIG. 6, which may help the heating element 206d become heated (via current delivery to the heating element 206d, as discussed herein) and/or may help prevent tissue and/or other material that is adjacent to the tissue being cut from being contacted by or heated by the heated heating element 206d.

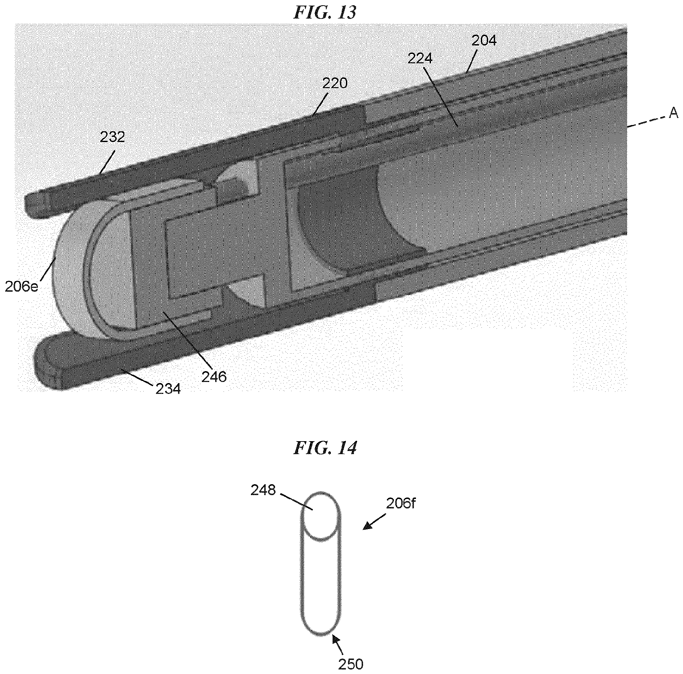

FIG. 13 illustrates another embodiment of the heating element 206 in which the heating element 206e has a U-shape. The embodiment of FIG. 13 is the same as the embodiment of FIG. 6 except that the connector 236 of FIG. 6 has a cylindrical shape while a connector 246 of FIG. 13 has a cube shape. Other connector shapes are possible, such as a rectangular box.

FIG. 14 illustrates another embodiment of the heating element 206 in which the heating element 206f has a cylindrical shape. The heating element 206f having the cylindrical shape can be, e.g., a wire or a rod. The heating element 206f includes a first substantially flat surface 248 that faces distally and a second, opposite substantially flat surface 250 that faces proximally.

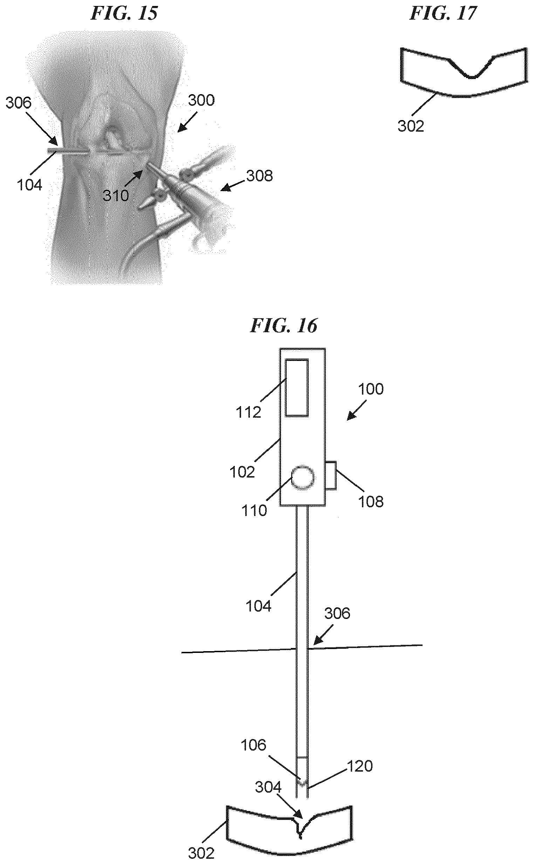

FIGS. 15 and 16 illustrate one embodiment of a method of using a thermal debriding tool in a surgical procedure to cut tissue of a patient. The method is described with respect to a meniscal debridement or repair procedure and the tool 100 of FIG. 3 but can be similarly performed in other surgical procedures and with other thermal debriding tools described herein.

The meniscal debridement or repair procedure includes the patient being prepped and sedated per typical prep and sedation procedures. The patient's knee 300 is draped and positioned as needed to allow for access to the patient's meniscus 302 that has a tear 304 to be treated. The meniscal tear 304 being treated in this illustrated embodiment is a radial tear, but other types of meniscal tears can be similarly treated, such as intrasubstance/incomplete tears, horizontal tears, bucket-handle tears, complex tears, and flap tears.

]As shown in FIGS. 15 and 16, the tool 100 in this illustrated embodiment is advanced into the patient and to the knee articular capsule through a lateral portal 306, such as a first skin incision. As shown in FIG. 15, an arthroscope 308 is advanced into the patient and to the knee articular capsule through a medial portal 310, such as a second skin incision, to provide for visualization and irrigation at the surgical site. The arthroscope 308 is positioned relative to the meniscus 302 to provide visualization of the meniscus 302 and the tear 304. Other surgical instrument(s) can be used instead of or in addition to the arthroscope 308 to provide visualization and irrigation. Portal locations other than the portal 306, 308 locations shown in FIG. 15 are possible and can be used as desired based on, e.g., surgeon preference, patient anatomy, whether the lateral meniscus or the medial meniscus is the meniscus tissue being treated, etc.

The tool 100 is maneuvered to contact and cut the meniscus 302 as described herein. Namely, the actuator 108 is actuated to heat the heating element 106, and the heated heating element 106 is moved along the meniscus 302, e.g., moved in a side-to-side motion, in the area of the tear 304 to cut the meniscus 302. FIG. 16 illustrates the meniscus 302 before the tool 100 cuts the meniscus 302. FIG. 17 illustrates the meniscus 302 after the tool 100 has cut the meniscus 302. As shown in FIG. 17, the tissue edge is smooth similar to the smooth tissue edge 16 of FIG. 2. As mentioned above, the actuator 108 can be actuated and unactuated one or more times to cut the meniscus 302, e.g., the actuator 108 actuated and then unactuated, the actuator 108 actuated again and then unactuated, etc.

After the tool 100 has cut the meniscus 302 as desired, the tool 100 and the arthroscope 308 can be removed from the patient and the portal 306, 308 incisions closed as needed.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces and subsequent reassembly. In particular, the device can be disassembled, and any number of the particular pieces or parts of the device can be selectively replaced or removed in any combination. Upon cleaning and/or replacement of particular parts, the device can be reconditioned for reuse after at least one use. Reconditioning can include any combination of the steps of disassembly of the device, followed by cleaning or replacement of particular pieces and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

One skilled in the art will appreciate further features and advantages of the arthroscopic medical implements and assemblies and methods based on the above-described embodiments. Accordingly, this disclosure is not to be limited by what has been particularly shown and described, except as indicated by the appended claims. All publications and references cited herein are expressly incorporated herein by reference in their entirety.

The present disclosure has been described above by way of example only within the context of the overall disclosure provided herein. It will be appreciated that modifications within the spirit and scope of the claims may be made without departing from the overall scope of the present disclosure.

## Claims

1. A surgical device, comprising:
a handle;
an elongate shaft extending distally from the handle, the shaft being configured to be advanced arthroscopically into a patient;
a conductive heating element at a distal end of the shaft, the heating element being configured to be heated such that contact of the heated heating element with tissue causes the heating element to cut the tissue;
a conductive lead operably coupled to the actuator and to the heating element, the conductive lead extending through the shaft; and
an actuator configured to be actuated and thereby cause a current to be delivered along the conductive lead to the heating element, thereby heating the heating element.

2. The device of claim 1, further comprising an insulative guard extending distally from the elongate shaft, the insulative guard including opposed distally-extending legs, the heating element being positioned between the distally-extending legs.

3. The device of claim 2, wherein the insulative guard is formed of a ceramic or of a plastic.

4. The device of claim 2, wherein the insulative guard is formed of a ceramic and a plastic, the plastic at least partially surrounds the ceramic, and the plastic has a lower conductivity than the ceramic.

5. The device of claim 1, further comprising a control circuit configured to cause the current delivered along the conductive lead to change during current delivery based on a resistance or temperature of the heating element.

6. The device of claim 1, wherein the shaft defines a longitudinal axis, and the heating element is U-shaped with opposed legs of the U-shape extending longitudinally and substantially parallel to the longitudinal axis.

7. The device of claim 1, wherein the heating element includes a substantially flat plate with a first edge of the plate facing distally and a second, opposite edge of the plate facing proximally.

8. The device of claim 1, wherein the actuation of the actuator is configured to cause the heating element to heat to a temperature in a range of about 500°C to about 1000°C.

9. The device of claim 1, wherein the conductive lead is formed of copper.

10. The device of claim 9, wherein the heating element is formed of a metal having a higher resistance than copper.

11. The device of claim 1, wherein an outer diameter of the shaft is in a range of about 2 mm to about 5 mm.

12. A surgical method, comprising:
arthroscopically advancing the surgical device of claim 1 to a knee of the patient; and
actuating the actuator, thereby causing the heating element to be heated and cut meniscus tissue.

13. A surgical method, comprising:
arthroscopically advancing the surgical device of claim 1 to one of a hip and a shoulder of the patient; and
actuating the actuator, thereby causing the heating element to be heated and cut tissue.

14. A surgical method, comprising:
positioning a conductive heating element of an arthroscopic surgical tool in contact with tissue of a patient, the heating element being at a distal end of an elongate shaft of the surgical tool; and
causing a current to be conducted through a conductive lead extending through the elongate shaft, thereby heating the heating element such that the heated heating element cuts the tissue.

15. The method of claim 14, further comprising, using a control circuit of the surgical tool, causing the current conducted through the conductive lead to change during conduction of the current based on a resistance or temperature of the heating element;
wherein heating the heating element includes heating the heating element to heat to a temperature in a range of about 500°C to about 1000°C.

16. The method of claim 14, wherein an insulative guard extends distally from the elongate shaft and includes opposed distally-extending legs;
the heating element is positioned between the distally-extending legs; and
the insulative guard protects tissue that is adjacent to the tissue being cut from being heated by the heated heating element.

17. The method of claim 14, wherein causing the current to be conducted through the conductive lead includes actuating an actuator of the surgical tool.

18. The method of claim 14, wherein the conductive lead is formed of copper; and
the heating element is formed of a metal having a higher resistance than copper.

19. The method of claim 14, further comprising advancing the surgical tool into the patient;
wherein the tissue includes meniscus tissue.

20. The method of claim 14, wherein the tissue is at one of a knee of the patient, a hip of the patient, and a shoulder of the patient.
